# EUROPEAN PATENT APPLICATION

(11) **EP 2 090 321 A1**
(43) Date of publication of application: **19.08.2009**
(21) Application number: 08002946.5
(22) Date of filing: 18.02.2008
(51) Int. Cl.: A61K 45/06

(54) **Health restorer comprising ascorbic acid, lactic acid, a pyruvate and magnesium oxide**

(71) Applicant: Galkina-Taylor, Olga Victorovna, Bournemouth Dorset BH9 2At (GB)
(72) Inventor: Galkina-Taylor, Olga Victorovna, Bournemouth Dorset BH9 2At (GB)

(57) **Abstract**

A health restorer comprising ascorbic acid, lactic acid, a pyruvate salt and magnesium oxide. The pyruvate salt is sodium pyruvate or potassium pyruvate. In addition the health restorer may include vitamin B3, vitamin B6 and chromium gluconate. Typical proportions will be ascorbic acid (1-3 parts); lactic acid (1-3 parts); sodium pyruvate (0.1- 1 part); magnesium oxide (1-2 parts); vitamin B3 (0.5-1 part); vitamin B6 (0.5-1 part); chromium gluconate (1-3 parts).

## Description

The present invention relates to a mixture of compounds for improving the health of patients and in particular, it helps them to fight disease.

Neurotoxins have been found in patients suffering from many disease. The presence of these neurotoxins is thought to be preventing normal brain function and in particular preventing the patient's immune system from fighting the disease to its full potential. It is believed that the neurotoxins originate from impaired metabolism after viral, bacterial, mycobacterial, parasitical and fungal infections.

Traces of neurotoxins have been found in patients suffering from leukaemia, chronic fatigue syndrome, multiple sclerosis and candida, as well as alcoholism, anxiety, stress and depression.

The object of the present invention is to provide a health restorer which prevents the formation of neurotoxins through rebalanced brain activity that will be able to control the immune and endocrine systems performance in a normal manner.

According to the present invention there is provided a health restorer including:
ascorbic acid;
lactic acid;
a pyruvate salt;
magnesium oxide.
While the pyruvate salt may be potassium pyruvate, normally it will be sodium pyruvate.

Typically the relative quantities are:
ascorbic acid 1-3 parts;
lactic acid 1-3 parts;
sodium pyruvate 0.1-1 part;
magnesium oxide 1-2 parts.
In addition the mixture may include:
vitamin B3 0.5-1 part;
vitamin B6 0.5-1 part;
chromium gluconate 1-3 parts.

The use of lactic acid prevents pyruvate being converted to lactic acid and stabilises the level of pyruvate. The ascorbic acid acts in the brain as an anti-oxidant to prevent the oxidation of neurotransmitters, including dopamine, serotonin and noradrenaline, at the synaptic cleft. The magnesium is involved in the production of neurotransmitters and the processes for their uptake by the synaptic terminals.

To assist in the understanding of the invention a specific embodiment thereof will now be described.

Typically, one dose of my treatment includes:
ascorbic acid 1 part
lactic acid 1 part
sodium pyruvate 0.125 parts
magnesium oxide 0.75 parts
   typically each part will be between 30mg and 200mg.

A patient who has been found to have traces of neurotoxins in his blood and urine can be treated with a capsule containing the above mixture. If the patient is then tested again after few days, usually no traces of neurotoxins can be detected or at least at substantially reduced levels.

The pyruvate salt used in the above example is sodium pyruvate. Potassium pyruvate can be used but has been found to be not so effective. It is likely that this is due to the formation of a complex preventing the pyruvate reaching the synaptic cleft.

The metabolites of ethanol, in particular in combination with dopamine, result in neurotoxins. The health restorer used on alcoholics includes the sodium pyruvate in combination with ascorbic acid, lactic acid and magnesium, with the addiction of vitamin B3 as alcoholics are often deficient in this vitamin. Thus, using this treatment, alcoholics have a much greater likelihood of ceasing their dependency on alcohol. The patients will take regular capsules of the treatment over a period of several weeks until they feel confident they can overcome their addiction.

Other additives, to the basic composition, can include vitamin B6 and chromium gluconate. Other vitamins and trace elements can also be added to the basic composition if the patient has these deficiencies.

In summary, my invention involves treating the patient with a mixture including sodium pyruvate. This is understood to rebalance the noradrenergic and serotonergic activities within the brain and may reduce the presence of neurotoxins and often eliminates them altogether. More importantly an improvement in the patient's health is seen. Furthermore anxiety and stress levels are likely to decrease with easing of depression, thus also assisting the patient in the achieving a state of well being.

## Claims

1. A health restorer including:
• ascorbic acid;
• lactic acid;
• a pyruvate salt;
• magnesium oxide.

2. A health restorer as claimed in claim 1, wherein the pyruvate salt is sodium pyruvate.

3. A health restorer as claimed in claim 1, wherein the pyruvate salt is potassium pyruvate.

4. A health restorer as claimed in claim 1, claim 2 or claim 3, wherein the relative quantities are:
• ascorbic acid 1-3 parts;
• lactic acid 1-3 parts;
• sodium pyruvate 0.1-1 part;
• magnesium oxide 1-2 parts.

5. A health restorer as claimed in any preceding claim, additionally including:
• vitamin B3 0.5-1 part;
• vitamin B6 0.5-1 part;
• chromium gluconate 1-3 parts.

6. A health restorer as claimed in any preceding claim comprising:
• ascorbic acid 1 part;
• lactic acid 1 part;
• sodium pyruvate 0.125 parts;
• magnesium oxide 0.75 parts.

7. A health restorer as claimed in claim 6, wherein each part is 30mg to 200mg.
